# EUROPÄISCHE PATENTSCHRIFT

(11) **EP 0 863 707 B1**
(45) Veröffentlichungstag und Bekanntmachung des Hinweises auf die Patenterteilung: **30.05.2001**
(21) Anmeldenummer: 96938136.7
(22) Anmeldetag: 07.11.1996
(51) Int. Cl.: A01N 47/36

(54) **5-ACYLAMINO-2-ALKOXYCARBONYLPHENYLSULFONYLHARNSTOFFE ALS SELEKTIVE HERBIZIDE**
5-ACYLAMINO-2-ALKOXYCARBONYL PHENYLSULPHONYL CARBAMIDES AS SELECTIVE HERBICIDES
5-ACYLAMINO-2-ALCOXYCARBONYLPHENYLSULFONYLUREES UTILISEES COMME HERBICIDES SELECTIFS

(30) Priorität: 01.12.1995 DE 19544743
(43) Veröffentlichungstag der Anmeldung: 16.09.1998
(73) Patentinhaber: Aventis CropScience GmbH, 65929 Frankfurt am Main (DE)
(72) Erfinder: SCHNABEL, Gerhard, D-63868 Gro wallstadt (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9604873
(87) Internationale Veröffentlichungsnummer: WO9720466

(56) Entgegenhaltungen:
- EP-A- 0 116 518
- WO-A-93/16998
- DE-A- 4 236 902
- DE-A- 4 415 049
- US-A- 4 547 215

## Beschreibung

Die Erfindung betrifft das technische Gebiet der Herbizide, insbesondere die Anwendung der Herbizide zur selektiven Bekämpfung von Unkräutern und Ungräsern in Nutzpflanzenkulturen.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen; siehe EP-A-1515, US-A-4892946, US-A-4981509, DE-A- 4322067, EP-A-116 518 (= US-A-4664695, US-A-4632695), DE-A-4236902 (WO 94/10154).

Viele der genannten Sulfonylharnstoffe lassen jedoch hinsichtlich der Selektivität zu wünschen übrig, d. h. sie können nicht in den zur Bekämpfung der Schadpflanzen erforderlichen Aufwandmengen eingesetzt werden, ohne die Kulturpflanzen in ungünstigem Maße zu schädigen.

Überraschenderweise wurde nun gefunden, daß bestimmte substituierte N-(Aminophenylsulfonyl)-N'-(pyrimidinyl- oder -triazinyl)-harnstoffe als selektive Herbizide vergleichsweise gut geeignet sind.

Gegenstand der vorliegenden Erfindung ist die Verwendung von Verbindungen der Formel (I) 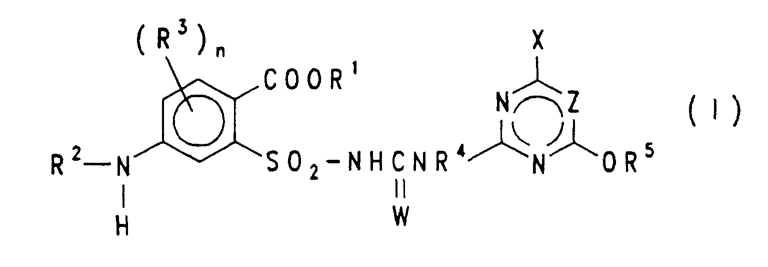 worin
- R¹: H, (C₁-C₆)-Alkyl, (C₂-C₆)-Alkenyl, (C₂-C₆)-Alkinyl, (C₃-C₆)-Cycloalkyl, wobei die vier letztgenannten Reste unsubstituiert oder durch einen oder mehrere Reste aus der Gruppe Halogen, CN, (C₁-C₃)-Alkoxy, (C₁-C₃)-Haloalkoxy, unsubstituiertes und substituiertes (C₃-C₆)-Cycloalkyl, (C₁-C₃)-Alkylthio, (C₁-C₃)-Alkylsulfinyl, (C₁-C₃)-Alkylsulfonyl, unsubstituiertes Phenyl, substituiertes Phenyl, unsubstituierter heterocyclischer Rest und substituierter heterocyclischer Rest und im Falle cyclischer Reste auch (C₁-C₄)-Alkyl und (C₁-C₄)-Haloalkyl substituiert sind, oder einen heterocyclischen Rest mit 3, 4, 5, 6 oder 7 Ringatomen und ein oder mehreren Atomen aus der Gruppe O, N und S als Heteroringatom, wobei der Rest unsubstituiert oder durch einen oder mehrere Reste aus der Gruppe Halogen, (C₁-C₃)-Alkyl und (C₁-C₃)-Haloalkyl substituiert ist,
- R²: einen Acylrest, ausgenommen Formyl,
- R³: Halogen, (C₁-C₆)-Alkyl, (C₁-C₆)-Alkoxy, (C₁-C₆)-Haloalkyl, (C₁-C₆)-Haloalkoxy, NO₂, CN, NH₂, (C₁-C₄)-Mono- oder Dialkylamino, und zwar jeweils unabhängig von anderen Resten R³, wenn n 2 oder 3 ist,
- R⁴: H oder (C₁-C₆)-Alkyl,
- R⁵: (C₁-C₆)-Alkyl oder (C₁-C₆)-Haloalkyl,
- x: ein Halogenatom aus der Gruppe F und Cl oder Halo-(C₁-C₃)-alkyl,
- n: 0, 1, 2 oder 3,
- W: ein Sauerstoffatom oder ein Schwefelatom und
- z: CH oder N bedeuten,
als selektive Herbizide zur Bekämpfung von Schadpflanzen in Nutzpflanzenkulturen.

In der deutschen Patentanmeldung P 4419259.2 (WO 95/32951) sind Salze von 5-Acylamino-2-alkoxycarbonylphenylsulfonylharnstoffen als wirkungsstarke Herbizide vorgeschlagen worden. Die Salze werden aus den entsprechenden freien Sulfonylharnstoffen durch Umsetzung mit einer Base hergestellt. Ein Teil der freien Sulfonylharnstoffe, die gemäß der deutschen Patentanmeldung P 4419259.2 (WO 95/32951) als Zwischenprodukte zur Herstellung der herbiziden Salze dienen, ist mit obengenannten Verbindungen der Formel (I) identisch. Es war jedoch bisher nicht bekannt, daß die Verbindungen der Formel (I) selbst als selektive Herbizide besonders geeignet sind.

In der deutschen Patentanmeldung Nr. 19510078.6 (WO 95/32950) sind bereits 5-Formylamino-2-alkoxycarbonylphenylsulfonylharnstoffen als wirkungsstarke selektive Herbizide vorgeschlagen worden.

In der genannten Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF2, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein heterocyclischer Rest oder Ring kann gesättigt, ungesättigt oder heteroaromatisch sein; er enthält ein oder mehrere Heteroringatome, vorzugsweise aus der Gruppe N, O und S; vorzugsweise ist er 5- oder 6-gliedrig und enthält 1, 2 oder 3 Heteroringatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(C₁-C₄)-Alkyl]-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Von besonderem Interesse sind erfindungsgemäße Verwendungen mit Verbindungen der Formel (I), worin
- R¹: (C₁-C₆)-Alkyl, (C₂-C₆)-Alkenyl, (C₂-C₆)-Alkinyl, (C₃-C₆)-Cycloalkyl, (C₄-C₈)-Cycloalkyl-(C₁-C₂)-alkyl, Phenyl-(C₁-C₆)-alkyl, wobei jeder der sechs letztgenannten Reste unsubstituiert oder durch einen oder mehrere Reste aus der Gruppe Halogen, wie F, Cl, Br und I, CN, OCH₃, OC₂H₅, OCF₃, SO₂CH₃ und im Falle cyclischer Reste auch (C₁-C₃)-Alkyl substituiert ist, oder einen Rest der Formeln A₁ bis A₇, insbesondere A₁, A₂, A₃, 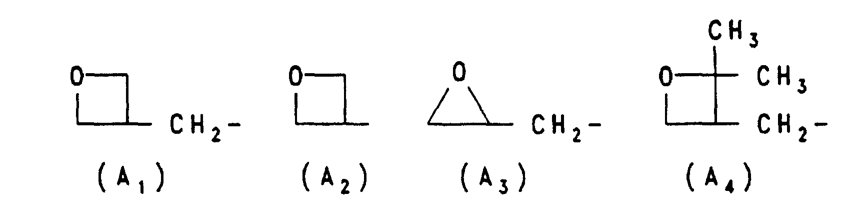 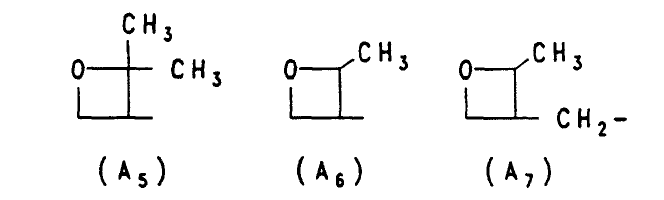
- R²: CO-R⁶, CO-OR⁷, CO-NR⁸R⁹, SO₂-R¹⁰,
- R³: Halogen, C₁-C₃-Alkyl, C₁-C₃-Alkoxy, C₁-C₃-Haloalkyl, C₁-C₃)-Haloalkoxy, NO₂, CN, NH₂, NHCH₃, N(CH₃)₂,
- R⁴: H oder (C₁-C₃)-Alkyl,
- R⁵: (C₁-C₃)-Alkyl oder (C₁-C₃)-Haloalkyl,
- R⁶: (C₁-C₆)-Alkyl, (C₂-C₆)-Alkenyl, (C₂-C₆)-Alkinyl, (C₃-C₆)-Cycloalkyl, wobei jeder der vier letztgenannten Reste unsubstituiert oder durch einen oder mehrere Reste aus der Gruppe Halogen, CN, (C₁-C₄)-Alkoxy, (C₁-C₄)-Haloalkoxy, Mono-(C₁-C₄)alkylamino, Di-(C₁-C₄)alkyl-amino, (C₁-C₄)-Alkylsulfonyl, (C₁-C₄)-Alkylsulfinyl, [(C₁-C₄)-Alkoxy]-carbonyl, Aminocarbonyl, Mono-[(C₁-C₄)-alkyl]-aminocarbonyl, Di-[(C₁-C₄)-alkyl]-aminocarbonyl, unsubstituiertes Phenyl und substituiertes Phenyl substituiert ist, oder Phenyl, das unsubstituiert oder substituiert ist,
- R⁷: einen Rest aus der Gruppe der für R⁶ möglichen Reste,
- R⁸ und R⁹: unabhängig voneinander H, (C₁-C₆)-Alkyl, (C₂-C₆)-Alkenyl, (C₂-C₆)-Alkinyl, wobei jeder der drei letztgenannten Reste unsubstituiert oder durch einen oder mehrere Reste aus der Gruppe Halogen, wie F, Cl und Br, und CN, (C₁-C₄)-Alkoxy, (C₁-C₄)-Haloalkoxy, Mono-(C₁-C₄)alkylamino, Di-(C₁-C₄)alkyl-amino, (C₁-C₄)-Alkylsulfonyl, (C₁-C₄)-Alkylsulfinyl, [(C₁-C₄)-Alkoxy]-carbonyl, Aminocarbonyl, Mono-(C₁-C₄)alkylaminocarbonyl und Di-[(C₁-C₄)-alkyl]-aminocarbonyl substituiert ist, oder
- R⁸ und R⁹: gemeinsam mit dem an sie gebundenen N-Atom einen unsubstituierten oder substituierten heterocyclischen Ring aus vier bis acht Ringatomen, der inklusive der Substituenten bis zu 18 C-Atome, vorzugsweise bis zu 12 C-Atomen enthält,
- R¹⁰: (C₁-C₅)-Alkyl, (C₂-C₅)-Alkenyl, wobei jeder der zwei letztgenannten Reste unsubstituiert oder durch einen oder mehrere Reste aus der Gruppe Halogen, (C₁-C₄)-Alkoxy, (C₁-C₄)-Haloalkoxy, Mono-(C₁-C₄)alkyl-amino und Di-(C₁-C₄)-alkyl-amino substituiert ist,
- n: 0 oder 1, vorzugsweise 0,
- W: O,
- x: F, Cl oder CF₃, vorzugsweise Cl,
- z: CH oder N, vorzugsweise CH bedeuten.

Bevorzugt sind erfindungsgemäße Verwendungen mit Verbindungen der Formel (I), worin
- R¹: (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₂)alkyl, Phenyl-(C₁-C₆)-alkyl, wobei jeder der sechs letztgenannten Reste unsubstituiert oder durch einen oder mehrere Reste aus der Gruppe Halogen, wie F, Cl, Br und I, und CN, OCH₃, OC₂H₅, OCF₃, SO₂CH₃ und im Falle cyclischer Reste auch (C₁-C₃)-Alkyl substituiert ist, oder einen Rest der Formeln A₁ bis A₇, insbesondere A₁, A₂, A₃, 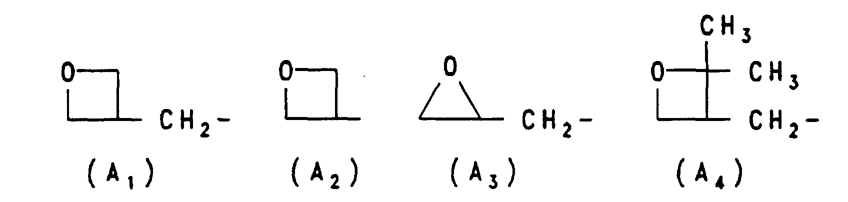 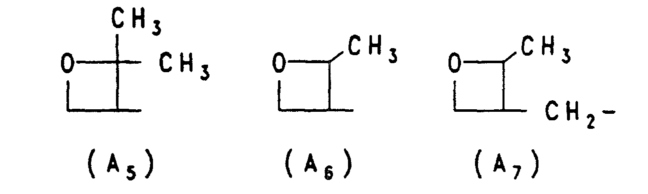
- R²: CO-R⁶, CO-OR⁷, CO-NR⁸R⁹, SO₂-R¹⁰,
- R³: Halogen, CH₃, C₂H₅, OCH₃, OC₂H₅, CF₃, CCl₃, OCF₃, OCHF₂ oder N(CH₃)₂,
- R⁴: H oder Methyl,
- R⁵: Methyl, Ethyl oder (C₁-C₂)-Haloalkyl,
- R⁶: (C₁-C₆)-Alkyl, (C₂-C₆)-Alkanyl, (C2-C₆)-Alkinyl, (C₃-C₆)-Cycloalkyl, wobei jeder der vier letztgenannten Reste unsubstitulert oder durch einen oder mehrere Reste aus der Gruppe F, Cl, Br, I, CN, OCH₃, OCF₃, N(CH₃)₂, SO₂CH₃, CO₂CH₃, CON(CH₃)₂ und Phenyl substituiert ist, oder Phenyl, das unsubstituiert oder bis zu dreifach durch gleiche oder verschiedene Reste aus der Gruppe Halogen, CH₃, C₂H₅, OCH₃, OC₂H₅, CF₃, C₂H₅Cl, OCHF₃, OCHF₂ substituiert ist,
- R⁷: einen Rest aus der Gruppe der für R⁶ möglichen Reste,
- R⁸ und R⁹: unabhängig voneinander H, (C₁-C₆)-Alkyl, (C₂-C₆)-Alkenyl, (C₂-C₆)-Alkinyl, wobei jeder der drei letztgenannten Reste unsubstituiert oder durch einen oder mehrere Reste aus der Gruppe F, Cl, Br, CN, OCH₃, OCF₃, N(CH₃)₂, SO₂CH₃, CO₂CH₃ und CON(CH₃)₂ substituiert ist, und
- R¹⁰: (C₁-C₄)-Alkyl oder (C₁-C₄)-Haloalkyl
bedeuten.

Besonders bevorzugt sind erfindungsgemäße Verwendungen mit Verbindungen der Formel (I), worin
- R¹: (C₁-C₄)-Alkyl, vorzugsweise Methyl oder Ethyl,
- R²: CO-R⁶, vorzugsweise CO-CH₃, CO-CH₂CH₃, Cyclopropyl-carbonyl, Isopropyl-carbonyl oder t-Butyl-carbonyl, insbesondere CO-CH₃, oder
- R²: CO-OR⁷, vorzugsweise COOCH₃, COOC₂H₅ und COOCH₂CH₂Cl, oder
- R²: CO-NR⁸R⁹, vorzugsweise CONH₂, CONHCH₃, CO-NH-C₂H₅, CON(CH₃)₂, oder
- R²: SO₂-R¹⁰, vorzugsweise SO₂CH₃, SO₂C₂H₅, SO₂CH₂F oder SO₂CH₂Cl,
bedeutet.

Die Verbindungen der Formel (I) können nach oder analog den in der obengenannten Literatur beschriebenen Verfahren hergestellt werden, insbesondere nach den in der deutschen Patentanmeldung Nr. 19510078.6 (WO 95/32950) für Formylderivate beschriebenen Verfahren. Anstelle des Formylrestes werden dann andere Acylreste verwendet und unter Einsatz von Acylierungsmitteln, die für die betreffenden Acylreste üblich sind, hergestellt.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Im direkten Vergleich mit strukturell nahen Sulfonylharnstoffherbiziden aus der 4,6-Dimethoxy- oder 4-Methoxy-6-methylpyrimidinyl oder -triazinylreihe oder mit anderen Aminophenylsulfonylharnstoffen zeigen die Verbindungen (I) überraschend verbesserte Selektivitäten. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchsstauchung, eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäß einzusetzenden Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, anderen Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.
Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff, versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Society of Chemistry, 1994, England, und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d.h. 2-Chlor-N,N-di-2-propenylacetamid; CDEC, d.h. Diethyldithiocarbaminsäure-2-chlorallylester; CGA 184927, d.h. 2-[4-[(5-Chlor-3-fluor-2-pyridinyl)-oxy]-phenoxy]-propansäure und -2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmedipham; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone; clomazon; dimethipin; dimetrasulfuron; cinosulfuron; dinitramine; dinoseb; dinoterb; diphenamid; dipropetryn; diquat; dithiopyr; diuron; DNOC; eglinazine-ethyl; EL 177, d.h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; F6285, d.h. 1-[5-(N-Methylsulfonyl)-amino-2,4-dichlorophenyl]-3-methyl-4-difluoromethyl-1,2,4-triazol-5-on; fenoprop; fenoxan; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d.h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate; quizalofop und dessen Esterderivate; quizalofop-ethyl; quizalofop-p-tefuryl; renriduron; dymron; S 275, d.h. 2-[4-Chlor-2-fluor-5-(2-propynyloxy)-phenyl]-4,5,6,7-tetrahydro-2H-indazol; S 482, d.h. 2-[7-Fluor-3,4-dihydro-3-oxo-4-(2-propynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d.h. 2-[[7-[2-Chlor-4-(trifluor-methyl)-phenoxy]-2-naphthalenyl)-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d.h. 5-Phenoxy-1-[3-(trifluormethyl)-phenyl]-1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden, auf dem die Pflanzen stehen oder in dem sie heranwachsen oder als Saat vorliegen, appliziert. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### Anwendungsbeispiele

Beispieie für die erfindungsgemäß einzusetztenden Verbindungen der Formel (I) sind in der folgenden Tabelle 1 aufgeführt. Folgende Abkürzungen werden in der Tabelle 1 verwendet:
Nr. = Beispielnummer
Fp. = Festpunkt (Schmelzpunkt) in °C
Me = Methyl
Et = Ethyl
Pr = ⁿPr = n-Propyl
ⁱPr = i-Propyl
^{c}Pr = Cyclopropyl
ⁿBu = n-Butyl
(Z) = Zersetzung

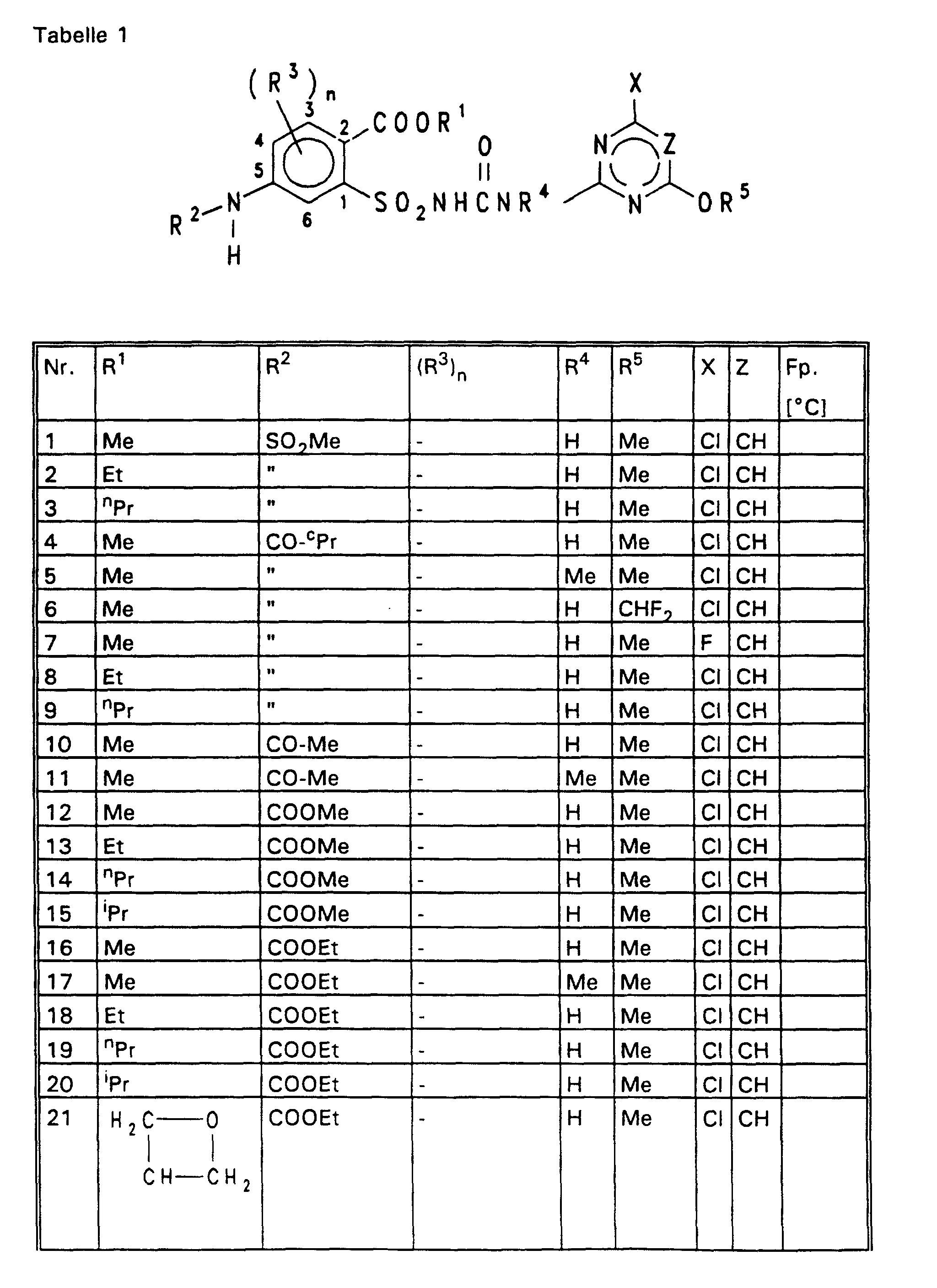

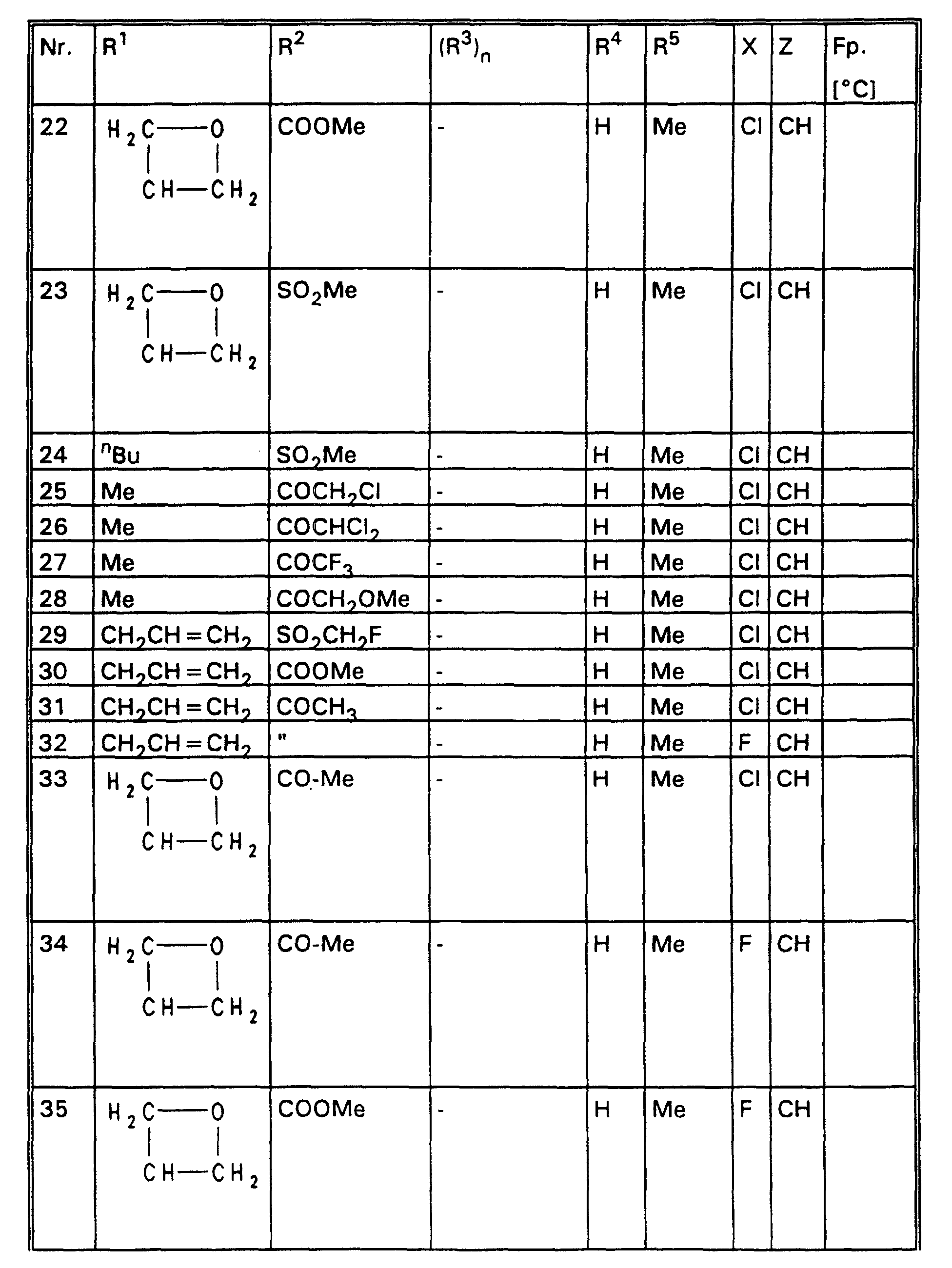

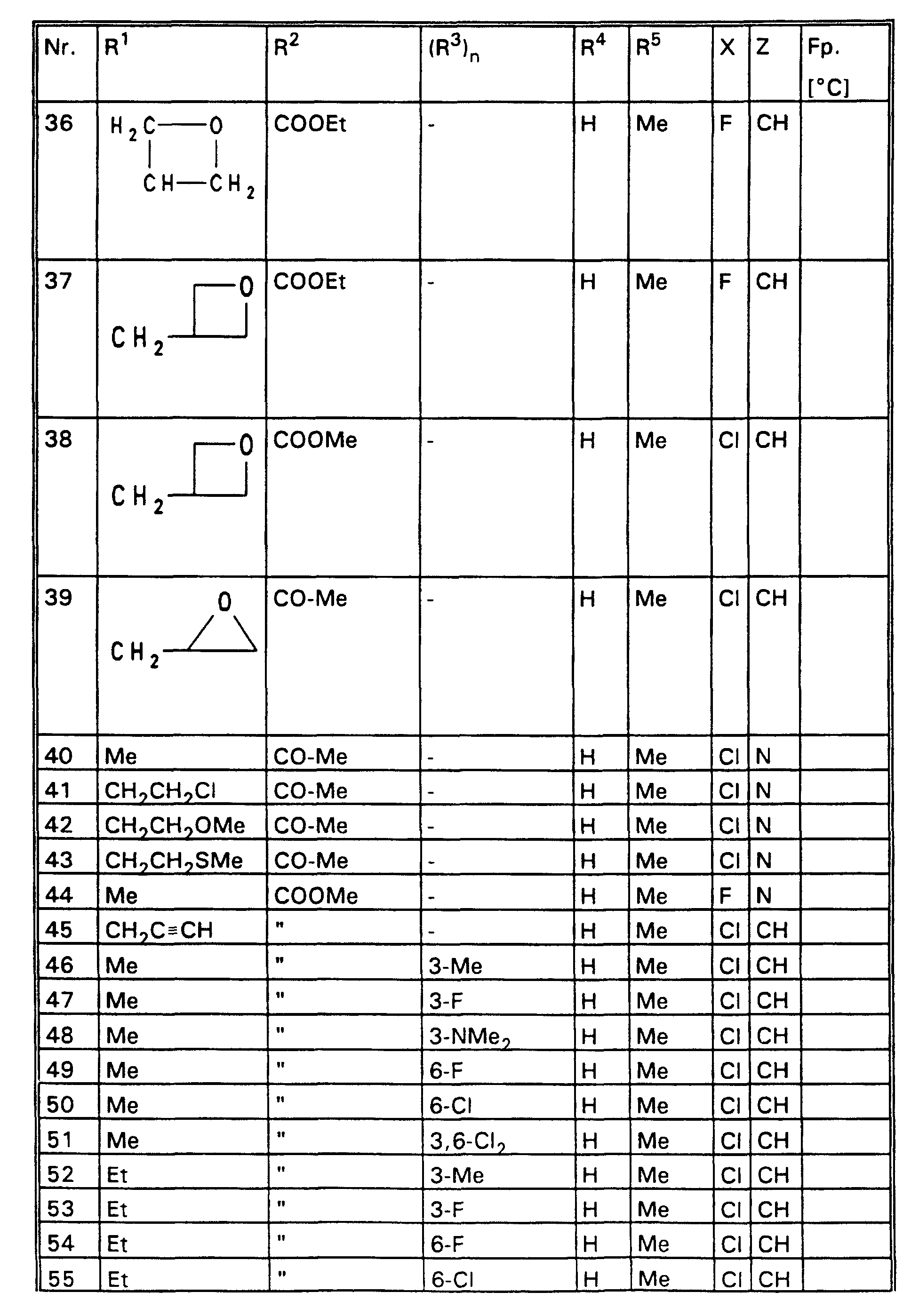

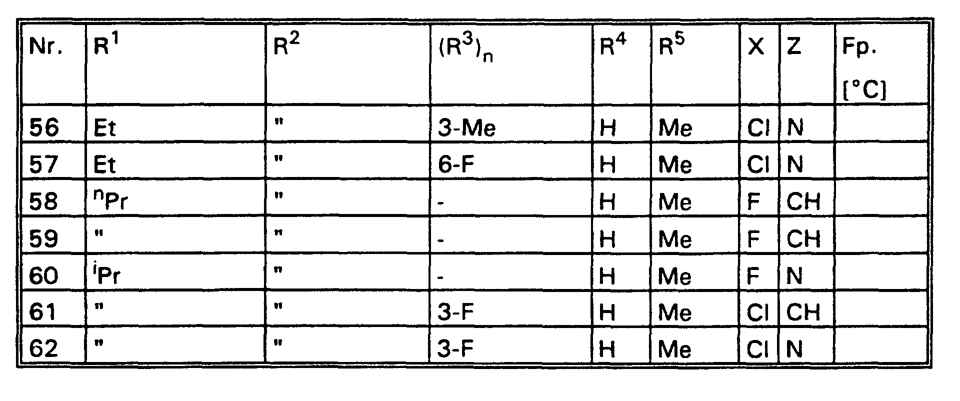

### Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit
   6 Gewichtsteilen Alkylphenolpolyglykolether (®Triton X 207),
   3 Gewichtsteilen Isotridecanolpolyglykolether (8 EO) und
   71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichtsteilen einer Verbindung der Formel (I), 75 Gewichtsteilen Cyclohexanon als Lösungsmittel und 10 Gewichtsteilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthylmethan-6,6'-disulfonsaures Natrium,
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise hat die Verbindung Nr. 12 aus der Tabelle 1 sehr gute herbizide Wirkung gegen Schadpflanzen wie Alopecurus myosuroides, Sinapis alba, Chrysanthemum segetum, Avena sativa, Stellaria media, Echinochlora crus-galli und Lolium multiflorum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt.

Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise hat die Verbindung Nr. 12 aus der Tabelle 1 sehr gute herbizide Wirkung gegen Schadpflanzen wie Alopecurus myosuroides, Sinapis alba, Stellaria media, Echinochloa crus-galli, Lolium multiflorum, Chrysanthemum segetum und Avena sativa im Nachauflaufverfahren bei einer Aufwandmenge von 0,3 kg und weniger Aktivsubstanz pro Hektar.

### 3. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vorund Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum-Hirsen, Mais oder Reis. Beispielsweise hat die Verbindung Nr. 12 aus Tabelle 1 sehr gute Selektivität im Nachauflaufverfahren in Kulturen wie Weizen Mais und Gerste. Die Verbindungen der Formel (I) weisen somit eine hohe Selektivität bei Anwendung zur Bekämpfung von unerwünschten Pflanzenwuchs in landwirtschaftlichen Kulturen auf.

## Patentansprüche

1. Verwendung von Verbindungen der Formel (I) 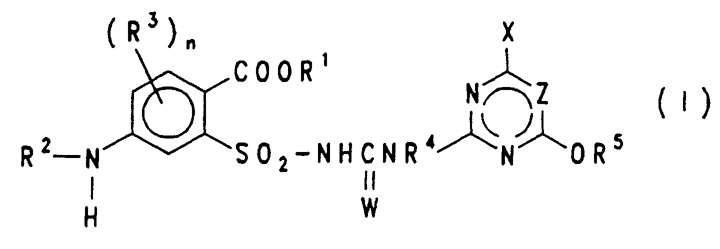 worin
R¹ H, (C₁-C₆)-Alkyl, (C₂-C₆)-Alkenyl, (C₂-C₆)-Alkinyl, (C₃-C₆)-Cycloalkyl, wobei die vier letztgenannten Reste unsubstituiert oder durch einen oder mehrere Reste aus der Gruppe Halogen, CN, (C₁-C₃)-Alkoxy, (C₁-C₃)-Haloalkoxy, unsubstituiertes oder substituiertes (C₃-C₆)-Cycloalkyl, (C₁-C₃)-Alkylthio, (C₁-C₃)-Alkylsulfinyl, (C₁-C₃)-Alkylsulfonyl, unsubstituiertes Phenyl, substituiertes Phenyl, unsubstituierter heterocyclischer Rest und substituierter heterocyclischer Rest und im Falle cyclischer Reste auch (C₁-C₄)-Alkyl und (C₁-C₄)-Haloalkyl substituiert sind, oder einen heterocyclischen Rest mit 3, 4, 5, 6 oder 7 Ringatomen und ein oder mehreren Atomen aus der Gruppe 0, N und S als Heteroringatom, wobei der Rest unsubstituiert oder durch einen oder mehrere Reste aus der Gruppe Halogen, (C₁-C₃)-Alkyl und (C₁-C₃)-Haloalkyl substituiert ist,
R² einen Acylrest, ausgenommen Formyl,
R³ Halogen, (C₁-C₆)-Alkyl, (C₁-C₆)-Alkoxy, (C₁-C₆)-Haloalkyl, (C₁-C₆)-Haloalkoxy, NO₂, CN, NH₂, (C₁-C₄)-Mono- oder Dialkylamino, und zwar jeweils unabhängig von anderen Resten R³, wenn n 2 oder 3 ist,
R⁴ H oder (C₁-C₆)-Alkyl,
R⁵ (C₁-C₆)-Alkyl oder (C₁-C₆)-Haloalkyl,
x ein Halogenatom aus der Gruppe F und Cl oder Halo-(C₁-C₃)-alkyl,
n 0, 1, 2 oder 3,
W ein Sauerstoffatom oder ein Schwefelatom und
Z CH oder N bedeuten,
als selektive Herbizide zur Bekämpfung von Schadpflanzen in Nutzpflanzenkulturen.

2. Verwendung nach Anspruch 1, dadurch gekennzeichnet, daß in Formel (I)
R¹ (C₁-C₆)-Alkyl, (C₂-C₆)-Alkenyl, (C₂-C₆)-Alkinyl, (C₃-C₆)-Cycloalkyl, (C₄-C₈)-Cycloalkyl-(C₁-C₂)-alkyl, Phenyl-(C₁-C₆)-alkyl, wobei jeder der sechs letztgenannten Reste unsubstituiert oder durch einen oder mehrere Reste aus der Gruppe Halogen, CN, OCH₃, OC₂H₅, OCF₃, SO₂CH₃ und im Falle cyclischer Reste auch (C₁-C₃)-Alkyl substituiert ist, oder einen Rest der Formeln A₁ bis A₇, insbesondere A₁, A₂, A₃, 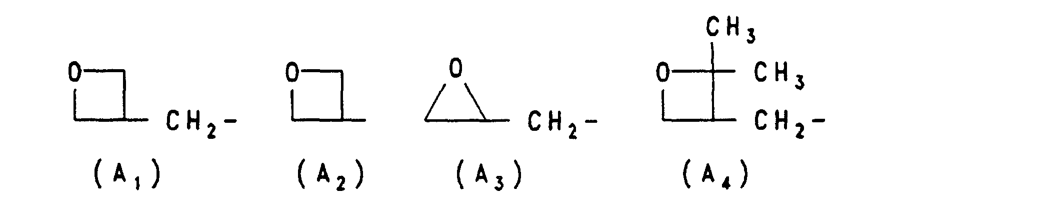 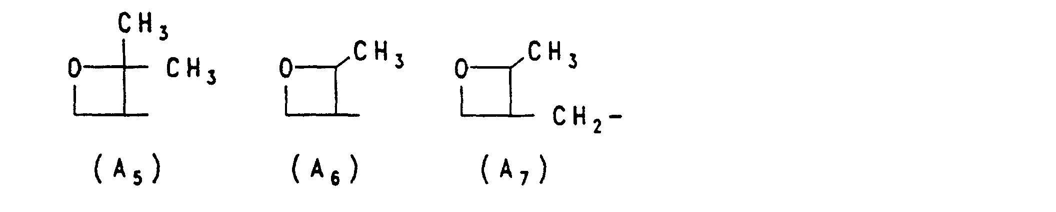
R² CO-R⁶, CO-OR⁷, CO-NR⁸R⁹, SO₂-R¹⁰,
R³ Halogen, C₁-C₃-Alkyl, C₁-C₃-Alkoxy, C₁-C₃-Haloalkyl, C₁-C₃)-Haloalkoxy, NO₂, CN, NH₂, NHCH₃, N(CH₃)₂,
R⁴ H oder (C₁-C₃)-Alkyl,
R⁵ (C₁-C₃)-Alkyl oder (C₁-C₃)-Haloalkyl,
R⁶ (C₁-C₆)-Alkyl, (C₂-C₆)-Alkenyl, (C₂-C₆)-Alkinyl, (C₃-C₆)-Cycloalkyl, wobei jeder der vier letztgenannten Reste unsubstituiert oder durch einen oder mehrere Reste aus der Gruppe Halogen, CN, (C₁-C₄)-Alkoxy, (C₁-C₄)-Haloalkoxy, Mono-(C₁-C₄)alkylamino, Di-(C₁-C₄)alkyl-amino, (C₁-C₄)-Alkylsulfonyl, (C₁-C₄)-Alkylsulfinyl, [(C₁-C₄)-Alkoxyl-carbonyl, Aminocarbonyl, Mono-[(C₁-C₄)-alkyl]-aminocarbonyl, Di-[(C₁-C₄)-alkyl]-aminocarbonyl, Phenyl und substituiertes Phenyl substituiert ist, oder Phenyl, das unsubstituiert oder substituiert ist,
R⁷ einen Rest aus der Gruppe der für R⁶ möglichen Reste,
R⁸ und R⁹ unabhängig voneinander H, (C₁-C₆)-Alkyl, (C₂-C₆)-Alkenyl, (C₂-C₆)-Alkinyl, wobei jeder der drei letztgenannten Reste unsubstituiert oder durch einen oder mehrere Reste aus der Gruppe Halogen, CN, (C₁-C₄)-Alkoxy, (C₁-C₄)-Haloalkoxy, Mono-(C₁-C₄)alkyl-amino, Di-(C₁-C₄)alkyl-amino, (C₁-C₄)-Alkylsulfonyl, (C₁-C₄)-Alkylsulfinyl, [(C₁-C₄)-Alkoxy]-carbonyl, Aminocarbonyl, Mono-(C₁-C₄)alkyl-aminocarbonyl und Di-[(C₁-C₄)-alkyl]-aminocarbonyl substituiert ist, oder
R⁸ und R⁹ gemeinsam mit dem an sie gebundenen N-Atom einen unsubstituierten oder substituierten heterocyclischen Ring aus vier bis acht Ringatomen, der inklusive der Substituenten bis zu 18 C-Atome enthält,
R¹⁰ (C₁-C₅)-Alkyl, (C₂-C₅)-Alkenyl, wobei jeder der zwei letztgenannten Reste unsubstituiert oder durch einen oder mehrere Reste aus der Gruppe Halogen, (C₁-C₄)-Alkoxy, (C₁-C₄)-Haloalkoxy, Mono-(C₁-C₄)alkyl-amino und Di-(C₁-C₄)-alkyl-amino substituiert ist,
n 0 oder 1,
W O,
x F, Cl oder CF₃ und
Z CH oder N bedeuten.

3. Verwendung nach Anspruch 1 oder 2, dadurch gekennzeichnet, daß
R¹ (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₂)alkyl, Phenyl-(C₁-C₆)-alkyl, wobei jeder der sechs letztgenannten Reste unsubstituiert oder durch einen oder mehrere Reste aus der Gruppe Halogen, CN, OCH₃, OC₂H₅, OCF₃, SO₂CH₃ und im Falle cyclischer Reste auch (C₁-C₃)-Alkyl substituiert ist, oder einen Rest der Formeln A₁ bis A₇, 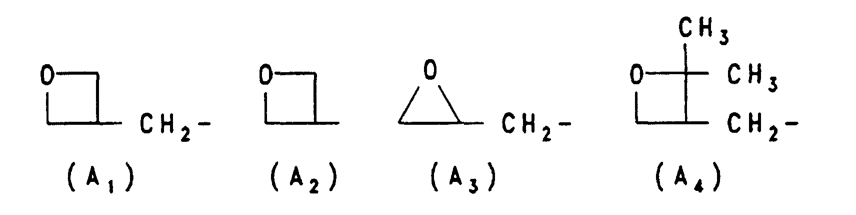 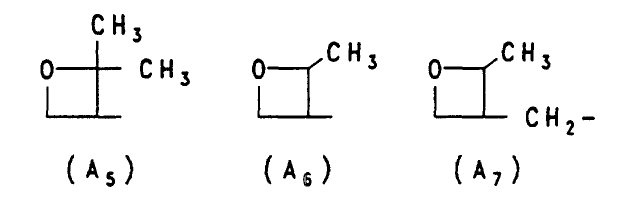
R² CO-R⁶, CO-OR⁷, CO-NR⁸R⁹, SO₂-R¹⁰,
R³ Halogen, CH₃, C₂H₅, OCH₃, OC₂H₅, CF₃, CCl₃, OCF₃, OCHF₂ oder N(CH₃)₂,
R⁴ H oder Methyl,
R⁵ Methyl, Ethyl oder (C₁-C₂)-Haloalkyl,
R⁶ (C₁-C₆)-Alkyl, (C₂-C₆)-Alkenyl, (C₂-C₆)-Alkinyl, (C₃-C₆)-Cycloalkyl, wobei jeder der vier letztgenannten Reste unsubstituiert oder durch einen oder mehrere Reste aus der Gruppe F, Cl, Br, I, CN, OCH₃, OCF₃, N(CH₃)₂, SO₂CH₃, CO₂CH₃, CON(CH₃)₂ und Phenyl substituiert ist, oder Phenyl, das unsubstituiert oder bis zu dreifach durch gleiche oder verschiedene Reste aus der Gruppe Halogen, CH₃, C₂H₅, OCH₃, OC₂H₅, CF₃, C₂H₅Cl, OCHF₃ und OCHF₂ substituiert ist,
R⁷ einen Rest aus der Gruppe der für R⁶ möglichen Reste,
R⁸ und R⁹ unabhängig voneinander H, (C₁-C₆)-Alkyl, (C₂-C₆)-Alkenyl, (C₂-C₆)-Alkinyl, wobei jeder der drei letztgenannten Reste unsubstituiert oder durch einen oder mehrere Reste aus der Gruppe F, Cl, Br, CN, OCH₃, OCF₃, N(CH₃)₂, SO₂CH₃, CO₂CH₃ und CON(CH₃)₂ substituiert ist, und
R¹⁰ (C₁-C₄)-Alkyl oder (C₁-C₄)-Haloalkyl
bedeuten.

4. Verwendung nach einem der Ansprüche 1 bis 3, dadurch gekennzeichnet, daß
R¹ (C₁-C₄)-Alkyl und
R² CO-CH₃, CO-CH₂CH₃, Cyclopropyl-carbonyl, Isopropyl-carbonyl, t-Butyl-carbonyl, COOCH₃, COOC₂H₅, COOCH₂CH₂Cl, CONH₂, CONHCH₃, CO-NH-C₂H₅, CON(CH₃)₂, SO₂CH₃, SO₂C₂H₅, SO₂CH₂F oder SO₂CH₂Cl
bedeutet.

5. Verwendung nach einem der Ansprüche 1 bis 4, dadurch gekennzeichnet, daß die Nutzpflanzenkultur ein Getreide ist.

6. Herbizides Mittel, dadurch gekennzeichnet, daß es mindestens eine Verbindung der Formel (I) nach einem der Ansprüche 1 bis 4 und im Pflanzenschutz übliche Formulierungshilfsmittel enthält.

7. Verfahren zur Bekämpfung von Schadpflanzen in Nutzpflanzenkulturen, dadurch gekennzeichnet, daß man ein wirksame Menge von mindestens einer Verbindung der Formel (I) nach einem der Ansprüche 1 bis 4 auf die Pflanzen, deren Pflanzensamen oder die Fläche, auf der die Pflanzen wachsen, appliziert.

8. Verfahren nach Anspruch 7, dadurch gekennzeichnet, daß die Nutzpflanzenkultur ein Getreide ist.

## Claims

1. The use of a compound of the formula (I) 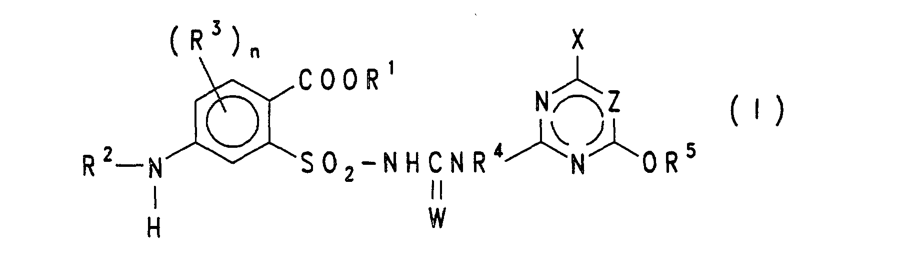 in which
R¹ is H, (C₁-C₆)-alkyl, (C₂-C₆)-alkenyl, (C₂-C₆)-alkynyl, (C₃-C₆)-cycloalkyl, where the last four radicals mentioned are unsubstituted or substituted by one or more radicals from the group consisting of halogen, CN, (C₁-C₃)-alkoxy, (C₁-C₃)-haloalkoxy, unsubstituted and substituted (C₃-C₆)-cycloalkyl, (C₁-C₃)-alkylthio, (C₁-C₃)-alkylsulfinyl, (C₁-C₃)-alkylsulfonyl, unsubstituted phenyl, substituted phenyl, an unsubstituted heterocyclic radical and a substituted heterocyclic radical and, in the case of cyclic radicals, also (C₁-C₄)-alkyl and (C₁-C₄)-haloalkyl, or a heterocyclic radical having 3, 4, 5, 6 or 7 ring atoms and one or more atoms from the group consisting of O, N and S as hetero ring atoms, the radical being unsubstituted or substituted by one or more radicals from the group consisting of halogen, (C₁-C₃)-alkyl and (C₁-C₃)-haloalkyl,
R² is an acyl radical, excluding formyl,
R³ is halogen, (C₁-C₆)-alkyl, (C₁-C₆)-alkoxy, (C₁-C₆)-haloalkyl, (C₁-C₆)-haloalkoxy, NO₂, CN, NH₂ or (C₁-C₄)-mono- or -dialkylamino, and in particular in each case independently of other radicals R³ if n is 2 or 3,
R⁴ is H or (C₁-C₆)-alkyl,
R⁵ is (C₁-C₆)-alkyl or (C₁-C₆)-haloalkyl,
x is a halogen atom from the group consisting of F and Cl or halo-(C₁-C₃)-alkyl,
n is 0, 1, 2 or 3,
w is an oxygen atom or a sulfur atom and
z is CH or N,
as a selective herbicide for controlling harmful plants in crops of useful plants.

2. The use as claimed in claim 1, wherein, in formula (I),
R¹ is (C₁-C₆)-alkyl, (C₂-C₆)-alkenyl, (C₂-C₆)-alkynyl, (C₃-C₆)-cycloalkyl, (C₄-C₈)-cycloalkyl- (C₁-C₂)-alkyl, phenyl-(C₁-C₆)-alkyl, where each of the last six radicals mentioned is unsubstituted or substituted by one or more radicals from the group consisting of halogen, CN, OCH₃, OC₂H₅, OCF₃, SO₂CH₃ and, in the case of cyclic radicals, also (C₁-C₃)-alkyl, or a radical of the formulae A₁ to A₇, in particular A₁, A₂ or A₃, 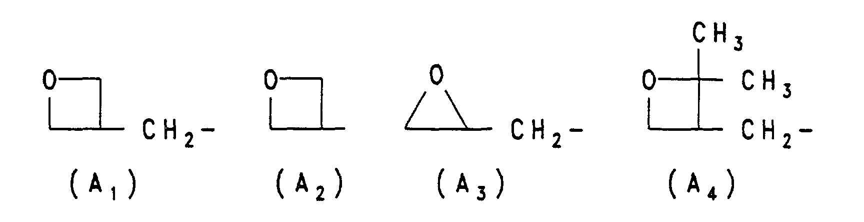 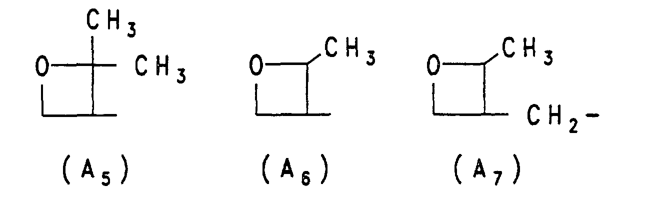
R² is CO-R⁶, CO-OR⁷, CO-NR⁸R⁹ or SO₂-R¹⁰,
R³ is halogen, (C₁-C₃)-alkyl, (C₁-C₃)-alkoxy, (C₁-C₃)-haloalkyl, (C₁-C₃)-haloalkoxy, NO₂, CN, NH₂, NHCH₃ or N (CH₃)₂,
R⁴ is H or (C₁-C₃)-alkyl,
R⁵ is (C₁-C₃)-alkyl or (C₁-C₃)-haloalkyl,
R⁶ is (C₁-C₆)-alkyl, (C₂-C₆)-alkenyl, (C₂-C₆)-alkynyl, (C₃-C₆)-cycloalkyl, where each of the last four radicals mentioned is unsubstituted or substituted by one or more radicals from the group consisting of halogen, CN, (C₁-C₄)-alkoxy, (C₁-C₄)-haloalkoxy, mono(C₁-C₄)-alkylamino, di-(C₁-C₄)-alkylamino, (C₁-C₄)-alkylsulfonyl, (C₁-C₄)-alkylsulfinyl, [(C₁-C₄)-alkoxy]carbonyl, aminocarbonyl, mono-[(C₁-C₄)-alkyl]aminocarbonyl, di-[(C₁-C₄)-alkyl]aminocarbonyl, phenyl and substituted phenyl, or phenyl, which is unsubstituted or substituted,
R⁷ is a radical from the group of radicals possible for R⁶,
R⁸ and R⁹ independently of one another are H, (C₁-C₆)-alkyl, (C₂-C₆)-alkenyl or (C₂-C₆)-alkynyl, where each of the last three radicals mentioned is unsubstituted or substituted by one or more radicals from the group consisting of halogen, CN, (C₁-C₄)-alkoxy, (C₁-C₄)-haloalkoxy, mono-(C₁-C₄)-alkylamino, di-(C₁-C₄)-alkylamino, (C₁-C₄)-alkylsulfonyl, (C₁-C₄)-alkylsulfinyl, [(C₁-C₄)-alkoxy]carbonyl, aminocarbonyl, mono-(C₁-C₄)-alkylaminocarbonyl and di-[(C₁-C₄)-alkyl]aminocarbonyl,or
R⁸ and R⁹, together with the N atom bonded to them, is an unsubstituted or substituted heterocyclic ring of four to eight ring atoms which, including the substituents, contains up to 18 carbon atoms,
R¹⁰ is (C₁-C₅)-alkyl or (C₂-C₅)-alkenyl, where each of the last two radicals mentioned is unsubstituted or substituted by one or more radicals from the group consisting of halogen, (C₁-C₄)-alkoxy, (C₁-C₄)-haloalkoxy, mono-(C₁-C₄)-alkylamino and di-(C₁-C₄)-alkylamino,
n is 0 or 1,
w is 0,
x is F, Cl or CF₃, and
z is CH or N.

3. The use as claimed in claim 1 or 2, wherein
R¹ is (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₂) -alkyl or phenyl-(C₁-C₆)-alkyl, where each of the last six radicals mentioned is unsubstituted or substituted by one or more radicals from the group consisting of halogen, CN, OCH₃, OC₂H₅, OCF₃, SO₂CH₃ and, in the case of cyclic radicals, also (C₁-C₃)-alkyl, or a radical of the formulae A₁ to A₇, 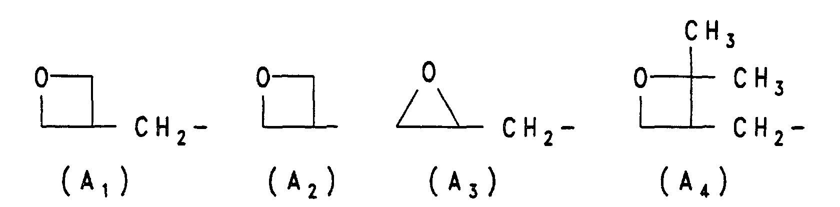 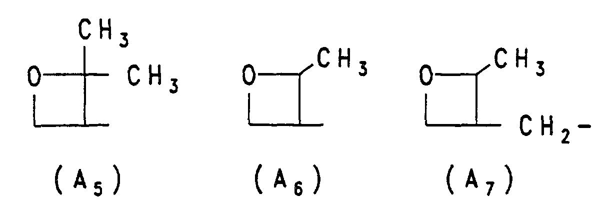
R² is CO-R⁶, CO-OR⁷, CO-NR⁸R⁹ or SO₂-R¹⁰,
R³ is halogen, CH₃, C₂H₅, OCH₃, OC₂H₅, CF₃, CCl₃, OCF₃, OCHF₂ or N(CH₃)₂,
R⁴ is H or methyl,
R⁵ is methyl, ethyl or (C₁-C₂)-haloalkyl,
R⁶ is (C₁-C₆)-alkyl, (C₂-C₆)-alkenyl, (C₂-C₆)-alkynyl, (C₃-C₆)-cycloalkyl, where each of the last four radicals mentioned is unsubstituted or substituted by one or more radicals from the group consisting of F, Cl, Br, I, CN, OCH₃, OCF₃, N(CH₃)₂, SO₂CH₃, CO₂CH₃, CON(CH₃)₂ and phenyl, or phenyl, which is unsubstituted or substituted up to three times by identical or different radicals from the group consisting of halogen, CH₃, C₂H₅, OCH₃, OC₂H₅, CF₃, C₂H₅Cl, OCHF₃ and OCHF₂,
R⁷ is a radical from the group of radicals possible for R⁶,
R⁸ and R⁹ independently of one another are H, (C₁-C₆)-alkyl, (C₂-C₆)-alkenyl or (C₂-C₆)-alkynyl, where each of the last three radicals mentioned is unsubstituted or substituted by one or more radicals from the group consisting of F, Cl, Br, CN, OCH₃, OCF₃, N(CH₃)₂, SO₂CH₃, CO₂CH₃ and CON(CH₃)₂, and
R¹⁰ is (C₁-C₄)-alkyl or (C₁-C₄)-haloalkyl.

4. The use as claimed in one of claims 1 to 3, wherein
R¹ is (C₁-C₄)-alkyl and
R² is CO-CH₃, CO-CH₂CH₃, cyclopropylcarbonyl, isopropylcarbonyl, t-butylcarbonyl, COOCH₃, COOC₂H₅, COOCH₂CH₂Cl, CONH₂, CONHCH₃, CO-NH-C₂H₅, CON(CH₃)₂, SO₂CH₃, SO₂C₂H₅, SO₂CH₂F or SO₂CH₂Cl.

5. The use as claimed in one of claims 1 to 4, wherein the crop of useful plants is a cereal.

6. A herbicidal composition which comprises at least one compound of the formula (I) as set forth in one of claims 1 to 4 and formulation auxiliaries customary in plant protection.

7. A method of controlling harmful plants in crops of useful plants, which comprises applying an active amount of at least one compound of the formula (I) set forth in one of claims 1 to 4 to the plants, plant seeds thereof or the area on which the plants grow.

8. The method as claimed in claim 7, wherein the crop of useful plants is a cereal.

## Revendications

1. Utilisation de composés de formule (I) 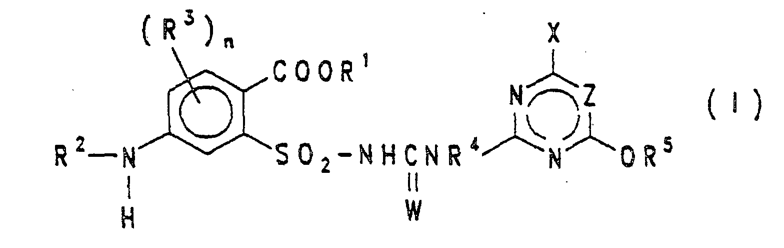 dans laquelle
R¹ représente un atome d'hydrogène, un groupe alkyle en C₁ à C₆, alcényle en C₂ à C₆, alcynyle en C₂ à C₆, cycloalkyle en C₃ à C₆, les quatre groupes cités en dernier étant non substitués ou substitués par un ou plusieurs groupes choisis parmi un atome d'halogène, CN, un groupe alcoxy en C₁ à C₃, halogénoalcoxy en C₁ à C₃, cycloalkyle en C₃ à C₆ non substitué ou substitué, alkylthio en C₁ à C₃, alkylsulfinyle en C₁ à C₃, alkylsulfonyle en C₁ à C₃, phényle non substitué, phényle substitué, radical hétérocyclique non substitué et radical hétérocyclique substitué et dans le cas de groupes cycliques aussi un groupe alkyle en C₁ à C₄ et halogénoalkyle en C₁ à C₄, ou représente un groupe hétérocyclique ayant 3, 4, 5, 6 ou 7 atomes de cycle et un ou plusieurs atomes choisis parmi O, N et S comme hétéroatomes de cycle, le groupe étant non substitué ou substitué par un ou plusieurs groupes choisi parmi un atome d'halogène, un groupe alkyle en C₁ à C₃ et halogénoalkyle en C₁ à C₃,
R² représente un groupe acyle, à l'exception du groupe formyle,
R³ représente un atome d'halogène, un groupe alkyle en C₁ à C₆, alcoxy en C₁ à C₆, halogénoalkyle en C₁ à C6, halogénoalcoxy en C₁ à C₆, NO₂, CN, NH₂, mono- ou dialkyle en (C₁ à C₄)-amino, et chacun indépendamment des autres groupes R³, lorsque n est 2 ou 3,
R⁴ représente un atome d'hydrogène ou un groupe alkyle en C₁ à C₆,
R⁵ représente un groupe alkyle en C₁ à C₆ ou halogénoalkyle en C₁ à C₆,
x représente un atome d'halogène choisi parmi F et C1 ou un groupe halogéno-alkyle en C₁ à C₃,
n est 0, 1, 2 ou 3,
w représente un atome d'oxygène ou un atome de soufre et
z représente CH ou N,
comme herbicides sélectifs pour la lutte contre les plantes nuisibles dans les cultures de plantes utiles.

2. Utilisation selon la revendication 1, caractérisée en ce que, dans la formule (I)
R¹ représente un groupe alkyle en C₁ à C₆, alcényle en C₂ à C₆, alcynyle en C₂ à C₆, cycloalkyle en C₃ à C₆, (cycloalkyle en C₄ à C₈)-alkyle en C₁ à C₂, phényl-alkyle en C₁ à C₆, chacun des six derniers groupes cités étant non substitué ou substitué par un ou plusieurs groupes choisis parmi les atomes d'halogène, CN, OCH₃, OC₂H₅, OCF₃, SO₂CH₃ et dans le cas de groupes cycliques aussi les groupes alkyle en C₁ à C₃, ou un groupe des formules A₁ à A₇, de préférence A₁, A₂, A₃, 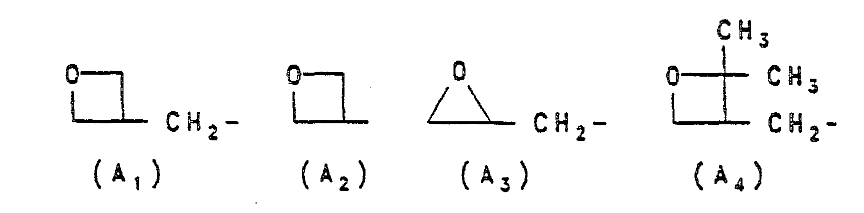 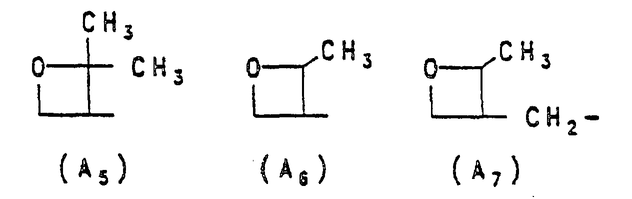
R² représente CO-R⁶, CO-OR⁷, CO-NR⁸R⁹, SO₂R¹⁰,
R³ représente un atome d'halogène, un groupe alkyle en C₁ à C₃, alcoxy en C₁ à C₃, halogénoalkyle en C₁ à C₃, halogéno-alcoxy en C₁ à C₃, NO₂, CN, NH₂, NHCH₃, N(CH₃)₂,
R⁴ représente un atome d'hydrogène ou un groupe alkyle en C₁ à C₃,
R⁵ représente un groupe alkyle en C₁ à C₃ ou halogéno-alkyle en C₁ à C₃,
R⁶ représente un groupe alkyle en C₁ à C₆, alcényle en C₂ à C₆, alcynyle en C₂ à C₆, cycloalkyle en C₃ à C₆, dans lesquels chacun des quatre derniers groupes cités est non substitué ou substitué par un ou plusieurs groupes choisis parmi un atome d'halogène, CN, un groupe alcoxy en C₁ à C₄, halogéno-alcoxy en C₁ à C₄, monoalkyle en (C₁ à C₄)-amino, dialkyle en (C₁ à C₄)-amino, alkyle en (C₁ à C₄)-sulfonyle, alkyle en (C₁ à C₄)-sulfinyle, [alcoxy en C₁ à C₄]-carbonyle, aminocarbonyle, mono-(alkyle en C₁ à C₄)-aminocarbonyle, di-[alkyle en C₁ à C₄]-aminocarbonyle, phényle et phényle substitué, ou phényle qui est non substitué ou substitué,
R⁷ représente un groupe choisi parmi les groupes possibles pour R⁶,
R⁸ et R⁹ représentent indépendamment l'un de l'autre H, un groupe alkyle en C₁ à C₆, alcényle en C₂ à C₆, alcynyle en C₂ à C₆, dans lesquels chacun des trois derniers groupes cités est non substitué ou substitué par un ou plusieurs groupes choisis parmi un atome d'halogène, CN, un groupe alcoxy en C₁ à C₄, halogéno-alcoxy en C₁ à C₄, monoalkyle en (C₁ à C₄)-amino, dialkyle en (C₁ à C₄)-amino, alkyle en (C₁ à C₄)-sulfonyle, alkyle en (C₁ à C₄)-sulfinyle, [alcoxy en C₁ à C₄]-carbonyle, aminocarbonyle, mono-(alkyle en C₁ à C₄)-aminocarbonyle et di-[alkyle en C₁ à C₄]-aminocarbonyle, ou
R⁸ et R⁹ représentent ensemble avec l'atome d'azote auquel ils sont liés un noyau hétérocyclique non substitué ou substitué constitué de quatre à huit atomes de cycle, qui contient inclusivement des substituants jusqu'à 18 atomes de carbone,
R¹⁰ représente un groupe alkyle en C₁ à C₅, alcényle en C₂ à C₅, dans lesquels chacun des deux derniers groupes cités est non substitué ou substitué par un ou plusieurs groupes choisis parmi un atome d'halogène, un groupe alcoxy en C₁ à C₄, halogéno-alcoxy en C₁ à C₄, monoalkyle en (C₁ à C₄)-amino, et dialkyle en (C₁ à C₄)-amino,
n est 0 ou 1,
W représente O,
x représente F, Cl ou CF₃,
z représente CH ou N.

3. Utilisation selon la revendication 1 ou 2, caractérisée en ce que
R¹ représente un groupe alkyle en C₁ à C₆, alcényle en C₂ à C₆, alcynyle en C₂ à C₆, cycloalkyle en C₃ à C₆, (cycloalkyle en C₃ à C₆)-alkyle en C₁ à C₂, phényl-alkyle en C₁ à C₆, chacun des six derniers groupes cités étant non substitué ou substitué par un ou plusieurs groupes choisis parmi les atomes d'halogène, CN, OCH₃, OC₂H₅, OCF₃, SO₂CH₃ et dans le cas de groupes cycliques aussi les groupes alkyle en C₁ à C₃, ou un groupe des formules A₁ à A₇, de préférence A₁, A₂, A₃, 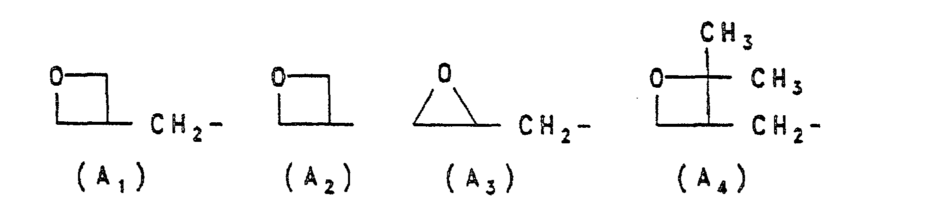 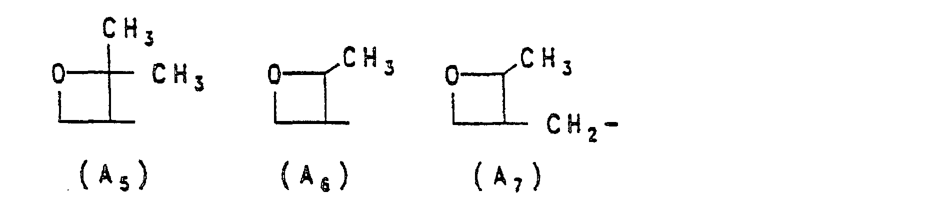
R² représente CO-R⁶, CO-OR⁷, CO-NR⁸R⁹, SO₂-R¹⁰,
R³ représente un atome d'halogène, CH₃, C₂H₅, OCH₃, OC₂H₅, CF₃, CCl₃, OCF₃, OCHF₂ ou N(CH₃)₂,
R⁴ représente un atome d'hydrogène ou un groupe méthyle,
R⁵ représente un groupe méthyle, éthyle ou halogéno-alkyle en C₁ à C₂,
R⁶ représente un groupe alkyle en C₁ à C₆, alcényle en C₂ à C₆, alcynyle en C₂ à C₆, cycloalkyle en C₃ à C₆, dans lesquels chacun des quatre derniers groupes cités est non substitué ou substitué par un ou plusieurs groupes choisis parmi F, Cl, Br, I, CN, OCH₃, OCF₃, N(CH₃)₂, SO₂CH₃, CO₂CH₃, CON(CH₃)₂ et phényle,
ou phényle qui est non substitué ou substitué jusqu'à trois fois par des groupes identiques ou différents choisis parmi un atome d'halogène, un groupe CH₃, C₂H₅, OCH₃, OC₂H₅, CF₃, C₂H₅Cl, OCHF₃, OCHF₂,
R⁷ représente un groupe choisi parmi les groupes possibles pour R⁶,
R⁸ et R⁹ représentent indépendamment l'un de l'autre H, un groupe alkyle en C₁ à C₆, alcényle en C₂ à C₆, alcynyle en C₂ à C₆, dans lesquels chacun des trois derniers groupes cités est non substitué ou substitué par un ou plusieurs groupes choisis parmi F, Cl, Br, CN, OCH₃, OCF₃, N(CH₃)₂, SO₂CH₃, CO₂CH₃ et CON(CH₃)₂, et
R¹⁰ représente un groupe alkyle en C₁ à C₄ ou halogéno-alkyle en C₁ à C₄.

4. Utilisation selon l'une des revendications 1 à 3, caractérisée en ce que
R¹ représente un groupe alkyle en C₁ à C₄ et
R² représente un groupe CO-CH₃, CO-CH₂CH₃, cyclopropylcarbonyle, isopropylcarbonyle, tert.-butylcarbonyle, COOCH₃, COOC₂H₅, COOCH₂CH₂Cl, CONH₂, COONHCH₃, CO-NH-C₂H₅, CON(CH₃)₂, SO₂CH₃, SO₂C₂H₅, SO₂CH₂F ou SO₂CH₂Cl.

5. Utilisation selon l'une des revendications 1 à 4, caractérisée en ce que les plantes utiles cultivées sont des céréales.

6. Agent herbicide, caractérisé en ce qu'il contient au moins un composé de formule (I) selon l'une des revendications 1 à 4 et des adjuvants de formulation usuels dans la protection des plantes.

7. Procédé de lutte contre les plantes nuisibles dans des cultures de plantes utiles, caractérisé en ce qu'on applique une quantité efficace d'au moins un composé de formule (I) selon l'une des revendications 1 à 4 sur les plantes, leurs semences ou la surface sur laquelle les plantes poussent.

8. Procédé selon la revendication 7, caractérisé en ce que les plantes utiles cultivées sont des céréales.
